# DEMANDE DE BREVET EUROPEEN

(11) **EP 4 763 253 A1**
(43) Date de publication de la demande: **24.06.2026**
(21) Numéro de dépôt: 25224335.7
(22) Date de dépôt: 17.12.2025
(51) Int. Cl.: A61M 11/04, A61M 15/00, B05B 11/00

(54) **DISPOSITIF MÉDICAL DE GÉNÉRATION D'AÉROSOL POUR L'ADMINISTRATION DE MÉDICAMENTS PAR INHALATION**

(30) Priorité: 20.12.2024 FR 2414910
(71) Demandeur: Assistance Publique - Hôpitaux de Paris, 75012 Paris (FR); Medinspire, 75012 Paris (FR)
(72) Inventeur: BERLIN, Ivan, 75012 Paris (FR); BIHAN, Kevin, 92290 Chatenay-Malabry (FR)
(74) Mandataire: Cabinet Camus Lebkiri

(57) **Abrégé**

L'ensemble médical (1) comprend :
- un réservoir (3) hermétique amovible, contenant un médicament liquide, et comportant un orifice de distribution obturable et une cheminée traversante,
- un plateau de chauffe (17), disposé sous le réservoir, comprenant une cavité de réception (18), ouverte sur le dessus et équipée d'une résistance chauffante,
- un arbre (13) vertical, entrainé en rotation par un moteur (14), sur lequel est monté le réservoir qui pivote entre une position de distribution et une position d'inhalation,
- une interface d'inhalation (6) buccale ou nasale.

En position de distribution, l'orifice de distribution est ouvert et surplombe la cavité de réception afin de la remplir de médicament. En position d'inhalation, l'orifice de distribution est fermé et la cheminée surplombe la cavité de réception. En chauffant, la résistance forme l'aérosol qui traverse la cheminée pour atteindre l'interface d'inhalation.

## Description

### DOMAINE TECHNIQUE DE L'INVENTION

La présente invention concerne un dispositif médical d'administration d'un médicament par inhalation, qui génère une quantité précise d'un aérosol de particules fines à partir d'un médicament sous forme liquide dont la qualité est préservée.

L'invention trouve des applications dans le domaine médical, en particulier, pour l'administration de médicaments par voie respiratoire jusqu'au niveau pulmonaire profond, en vue d'un effet systémique ou local.

### ARRIERE-PLAN TECHNOLOGIQUE DE L'INVENTION

La voie inhalée est une voie intéressante d'administration des médicaments, car elle présente l'avantage d'être non-invasive, d'assurer une très bonne biodisponibilité en raison d'une surface d'absorption alvéolo-capillaire étendue en contact direct avec la circulation pulmonaire veineuse (sang artériel), d'éviter une métabolisation précoce indésirable par voie gastro-entérique et hépatique, et de présenter un délai d'apparition de l'effet thérapeutique très court.

La voie inhalée est généralement utilisée pour des actions locales, essentiellement bronchiques, via un aérosol généré par des dispositifs médicaux adaptés. Un aérosol est une suspension dans un gaz ou mélange gazeux (air) d'un ensemble de particules solides ou liquides de faible diamètre.

Les dispositifs médicaux utilisés actuellement pour cela sont principalement des nébuliseurs, qui génèrent un aérosol de particules liquides au moyen d'un flux d'air ou d'oxygène sous pression qui traverse une chambre où se trouve le médicament liquide et l'entraine sous forme d'une brume (aérosol) qui est inhalée par le patient à l'aide d'un masque ou d'un embout buccal ou nasal.

Cette brume, ou aérosol, a l'avantage de faire arriver le principe actif rapidement sur les sites d'action (bronches) avec un passage relativement faible du principe actif dans la circulation générale. Cependant, les particules d'aérosol délivrées par un nébuliseur sont en moyenne comprises entre 3 et 5 µm de diamètre, ce qui limite la quantité de principe actif atteignant les alvéoles bronchiques. En effet, une grande partie du principe actif (environ 80-85 %) reste dans la cavité buccale et l'œsophage et seulement environ 20% des particules arrivent dans les bronches (cibles et sites d'action). Parmi ces 20%, seule une infime partie, correspondant aux particules les plus fines de diamètre inférieur à 2 µm, atteint le poumon profond (bronchioles et alvéoles) et/ou contribue aux effets systémiques, qui ne sont donc pas des cibles thérapeutiques réalistes pour ces médicaments. Cette modalité d'administration de médicaments par nébuliseur classique doit donc être réservée à des médicaments ciblant les voies respiratoires hautes (s'étendant de la cavité nasale jusqu'aux bronches de large diamètre) mais est inadaptée si l'on souhaite une action au niveau du poumon profond ou une action systémique des médicaments.

Les nébuliseurs présentent en outre l'inconvénient d'être bruyants, encombrants, couteux et contraignants en terme d'utilisation, car ils nécessitent un entretien régulier et l'intervention d'un personnel formé pour préparer le médicament à administrer. Ils sont donc peu appropriés pour un usage en ambulatoire et en autonomie par le patient lui-même.

D'autres dispositifs médicaux utilisés pour l'administration par voie inhalée sont les aérosols-doseurs (ou sprays) et les inhalateurs de poudres, généralement utilisés dans le traitement de l'asthme. Bien que ces dispositifs soient simples d'utilisation et parfaitement adaptés à une utilisation en ambulatoire, ils présentent l'inconvénient - à l'instar des nébuliseurs - de générer des aérosols avec des particules de dimensions élevées (diamètre supérieur à 2 µm) qui restent, pour leur majorité, dans le dispositif médical lui-même ou la sphère buccale et l'œsophage. Par ailleurs, ces dispositifs présentent l'inconvénient de nécessiter une parfaite coordination main-inhalation, une inspiration forcée profonde retenue et la nécessité d'un lavage buccal après utilisation.

Un dispositif médical plus avantageux a été décrit dans la demande WO 2023/166025A1. Ce dispositif comprend un boîtier dans lequel est monté un réservoir hermétique renfermant le médicament liquide, un dispositif d'aérosolisation du médicament qui génère un aérosol micro-particulé contenant des particules de diamètre inférieur à 2 µm dispersées dans l'air, un embout buccal en liaison fluidique avec le réservoir et par lequel l'aérosol peut être inhalé par le patient, et une unité de commande qui traite des données relatives à l'administration du médicament et contrôle la posologie du médicament inhalé par le patient.

Le dispositif d'aérosolisation comprend un élément chauffant mobile qui vient s'engager dans le réservoir et chauffe le médicament pour obtenir son aérosolisation. Cet élément chauffant est logé dans un fourreau creux permettant le passage de l'air et comprend un fil de résistance électrique entouré par une couche de coton qui forme une paroi d'interface poreuse entre le liquide contenu dans le réservoir et le fil de résistance. Le médicament liquide absorbé dans la couche de coton est chauffé par le fil de résistance, créant des microgouttelettes qui sont entrainées par l'air traversant le fourreau et forment ainsi l'aérosol.

Le fait de chauffer la solution médicamenteuse pour produire l'aérosol permet avantageusement de réduire la taille des particules dispersées dans l'air jusqu'à un diamètre inférieur à 2 µm. Le médicament aérosolisé peut ainsi pénétrer beaucoup plus profondément dans les voies respiratoires lorsqu'il est inhalé par le patient et atteindre les bronchioles et alvéoles pulmonaires en quantité suffisante pour une action locale broncho-alvéolaire ou avec une biodisponibilité suffisante pour une action systémique efficace et rapide, ce qui le rend utilisable dans des indications diverses.

Bien que prometteur, ce dispositif antérieur d'administration de médicaments par voie inhalée présente plusieurs désavantages.

Premièrement, comme l'élément chauffant mobile pénètre dans le réservoir pour chauffer le médicament, l'ensemble du médicament contenu dans le réservoir est chauffé à chaque utilisation. De plus, lorsque le réservoir contient plusieurs doses, l'ensemble du médicament est chauffé à chaque utilisation et donc de multiple fois avant d'être inhalé. Or, ces opérations de chauffe globales et répétées sont susceptibles de causer une dégradation des médicaments pouvant réduire leur efficacité thérapeutique.

En outre, le dosage précis du médicament administré au patient est difficile à réaliser. En effet, lorsque l'élément chauffant est engagé dans le réservoir, la couche de coton s'imprègne de médicament en continu au fur et à mesure de sa consommation dans l'aérosol produit. La quantité précise de liquide utilisée est donc difficilement contrôlable. De plus, lorsque l'appareil est utilisé plusieurs fois successivement, une fraction de la dose précédente reste imprégnée sur la paroi de coton et se mélange à la nouvelle dose, rendant le dosage approximatif.

Par ailleurs, la paroi de coton qui est traversée par la solution médicamenteuse représente une source de contamination potentielle non négligeable. En effet, sa composition n'est pas entièrement connue et difficilement maîtrisable. Elle contient souvent de nombreuses impuretés qui sont susceptibles de polluer le médicament.

De plus, lorsque le dispositif n'a pas été utilisé pendant longtemps ou est utilisé pour administrer plusieurs médicaments différents, une fraction de la dose précédente, ancienne ou de nature différente, peut être imprégnée sur la paroi de coton et peut contaminer ou polluer la nouvelle dose de médicament. Le coton doit donc être changé régulièrement par le patient pour éviter de tels désagréments. Or il s'agit d'une opération relativement délicate, qui n'est pas toujours réalisée par les patients malgré les préconisations, et qui si elle est mal faite peut induire un risque d'infection pulmonaire.

Ce dispositif antérieur ne permet donc pas de garantir l'administration d'une dose précise de médicament de qualité préservée et dans des conditions stériles.

Par ailleurs, on connait les dispositifs de vapotage (cigarette électronique), qui sont peu coûteux, très simples d'utilisation et qui génèrent également un aérosol inhalé par l'utilisateur à partir d'un liquide chauffé. Cependant, ces dispositifs de vapotage ne sont pas des dispositifs médicaux et ne sont pas du tout adapté à l'administration de médicaments. Ils ne sont pas sécurisés et la quantité inhalée n'est pas mesurée. Ils ne permettent pas la délivrance d'une dose précise et ne respectent pas la règlementation des dispositifs médicaux. Ils sont donc incompatibles avec un usage thérapeutique. En outre, ils comportent également une couche de coton servant d'interface poreuse entre le liquide à aérosoliser et l'élément de chauffe. Comme aucun système de retrait du système de chauffe avec le coton n'est prévu dans ces dispositifs, le risque de contamination et de dégradation du principe actif est encore nettement supérieur à celui engendré par le dispositif précédent.

Il existe donc un besoin non satisfait d'un dispositif médical d'administration par voie inhalée permettant l'auto-administration sous la forme d'un aérosol micro-particulé d'une dose précise d'un médicament liquide dont la qualité est préservée.

### RESUME DE L'INVENTION

L'invention a pour but de répondre à ce besoin.

Pour cela, un premier aspect de l'invention enseigne un ensemble comprenant un dispositif médical d'administration d'un médicament par inhalation et un réservoir hermétique amovible contenant ledit médicament sous une forme liquide, le dispositif médical générant un aérosol inhalable par le patient par élévation de la température du médicament.

Ce dispositif médical comprend :
- un bâti renfermant un logement dans lequel est placé le réservoir,
- une interface d'inhalation de l'aérosol,
- une unité de commande, contrôlant le fonctionnement du dispositif médical.

Selon l'invention, ce dispositif médical comprend en outre :
- un arbre vertical, dont une extrémité dépasse dans le logement et sur laquelle le réservoir est monté,
- un moteur qui entraine en rotation l'arbre vertical et qui, par l'intermédiaire de cet arbre vertical, fait pivoter le réservoir entre au moins une position de distribution et au moins une position d'inhalation,
- un plateau de chauffe, disposé sous le logement, qui comprend au moins une cavité de réception du médicament, comportant une ouverture supérieure et équipée d'une résistance chauffante permettant d'élever la température à l'intérieur de la cavité de réception.

D'autre part, le réservoir comporte :
- une paroi inférieure dans laquelle est ménagée un orifice de distribution, permettant l'écoulement du médicament hors du réservoir, ledit orifice de distribution se trouvant au-dessus de l'ouverture supérieure de la cavité de réception lorsque le réservoir est en position de distribution,
- un élément d'obturation, qui bouche l'orifice de distribution lorsque le réservoir n'est pas en position de distribution et le laisse libre lorsque le réservoir est en position de distribution, et
- au moins une cheminée traversante, qui, lorsque le réservoir est en position d'inhalation, se trouve au-dessus de l'ouverture supérieure de la cavité de réception, mettant la cavité de réception en communication fluidique avec l'interface d'inhalation.

Lorsqu'une dose de médicament doit être administrée, l'unité de commande provoque la rotation du réservoir, par l'intermédiaire du moteur qui fait tourner l'arbre vertical sur lequel le réservoir est monté, jusqu'à ce qu'il atteigne une position de distribution.

Dans cette position, l'élément d'obturation libère l'orifice de distribution du réservoir qui se trouve juste au-dessus de l'ouverture supérieure de la cavité de réception. Le médicament liquide s'écoule du réservoir et remplit la cavité de réception servant d'unité de chauffe.

Lorsque la cavité de réception est remplie, l'unité de commande fait pivoter à nouveau le réservoir rotatif jusqu'à ce qu'il atteigne une position d'inhalation. Dès que le réservoir quitte la position de distribution, l'élément d'obturation vient boucher l'orifice de distribution du réservoir ce qui empêche tout écoulement indésirable.

Lorsque le réservoir atteint une position d'inhalation, la cheminée se trouve au-dessus de la cavité de réception. L'unité de commande active alors la résistance chauffante de la cavité de réception qui chauffe la dose de médicament contenue dans la cavité de réception (uniquement celle-ci et non l'ensemble du réservoir). Cette élévation de température permet l'aérosolisation de cette dose de médicament, qui s'élève au-dessus de la cavité de réception, traverse la cheminée et atteint l'interface d'inhalation à travers laquelle elle est administrée au patient.

Ces opérations peuvent être répétées autant de fois que nécessaire si une quantité plus importante de médicament doit être administrée au patient.

Avec le dispositif selon l'invention, le dosage du médicament est ainsi réalisé automatiquement et de façon très précise grâce au remplissage de la cavité de réception, la dose inhalée correspondant exactement à un ou plusieurs volumes de cette cavité de réception.

Comme l'aérosol est obtenu par élévation de la température du médicament, il est formé de particules de très petite taille, dont la majorité présente un diamètre inférieur à 2 µm. Le médicament aérosolisé peut ainsi pénétrer très profondément dans les voies respiratoires lorsqu'il est inhalé par le patient, et avoir une diffusion importante dans les voies respiratoires basses jusqu'au niveau alvéolaire.

Avantageusement, l'ensemble selon l'invention permet de chauffer uniquement la quantité précise de médicament qui doit être administrée et non la totalité du médicament contenu dans le réservoir évitant ainsi un risque de surdosage du médicament ou sa dégradation prématurée. Le médicament non utilisé restant dans le réservoir est donc préservé et ne subit pas inutilement plusieurs cycles de chauffe comme dans les dispositifs de l'art antérieur.

En outre, dans le dispositif selon l'invention, la résistance chauffante est dépourvue de matériau d'imprégnation, tel que du coton ou de la silice par exemple. Comme le médicament n'est pas imprégné sur un matériau absorbant de type coton ou autre, pouvant constituer une source de contamination ou de pollution potentielle. La qualité du médicament est là encore préservée. Cet affranchissement du coton ou d'autres systèmes d'imprégnation de liquide permet également de s'assurer de la quantité de médicament délivrée en évitant une rétention du médicament issue des administrations précédentes.

De plus, ce dispositif permet de sécuriser l'administration du médicament tant en termes de contrôle des administrations et des posologies, qu'en termes de manipulation et de maintenance, car le patient n'a accès qu'au réservoir amovible qui est hermétiquement clos et qu'il doit simplement mettre en place dans le logement, puis retirer. Il n'a pas accès au médicament situé à l'intérieur du réservoir dans un milieu stérile et l'ajustement des posologies est contrôlé et paramétré automatiquement par le dispositif lui-même. Le patient n'a aucune opération de maintenance délicate à réaliser (comme le remplacement du coton par exemple), qui dans le cas contraire pourrait être oubliée ou mal faite.

Enfin, le dispositif selon l'invention est portatif et ergonomique (c'est-à-dire de dimensions suffisamment réduites pour permettre une utilisation manuelle facile), et permet une auto-administration par le patient en ambulatoire.

Avantageusement, le plateau de chauffe peut comprendre plusieurs, et préférentiellement quatre, cavités de réception, équipées chacune d'une résistance chauffante. Le réservoir comporte alors un nombre de cheminées traversantes correspondant au nombre de cavités de réception, lesdites cheminées traversantes se trouvant chacune au-dessus de l'ouverture supérieure de l'une des cavités de réception lorsque le réservoir est en position d'inhalation, mettant ladite cavité de réception en communication fluidique avec l'interface d'inhalation.

Dans ce cas, il est possible de remplir plusieurs cavités de réception en enchaînant plusieurs opérations de distribution, par simple rotation du réservoir le plaçant successivement en position de distribution au-dessus de chacune des cavités de réception à remplir. Comme l'élément d'obturation rebouche l'orifice de distribution dès que le réservoir quitte l'une des positions de distribution, aucun écoulement indésirable ne se produit entre les opérations de distribution.

Lorsque toutes les cavités de réception souhaitées sont remplies, l'unité de commande fait pivoter à nouveau le réservoir rotatif jusqu'à ce qu'il atteigne une position d'inhalation. Dans cette position, chacune des cavités de réception est surmontée par une cheminée traversante qui la met en communication fluidique avec l'interface d'inhalation.

L'unité de commande active alors les résistances chauffantes des cavités de réception préalablement remplies, ce qui chauffe ; de préférence jusqu'à totale aérosolisation, les quantités de solutions médicamenteuses distribuées qu'elles contiennent. Une fois aérosolisées, ces aérosols contiennent de fines particules de médicament qui s'élèvent au-dessus de ces cavités de réception, traversent les cheminées et aboutissent à l'interface d'inhalation qui permet leur inhalation par le patient.

Il est ainsi possible de contrôler finement la posologie du médicament à administrer et de l'adapter par exemple en fonction de la pathologie, du patient, de la concentration du médicament ou encore de la phase du traitement. En effet, un nombre quelconque de cavité de réception, compris entre un et le nombre total de cavités, peut être rempli à chaque cycle, ce qui permet de modifier le volume total de médicament transformé en aérosol (correspondant à la dose) par prise.

Un tel mode de réalisation permet en outre d'aérosoliser une grande quantité de médicament avec un temps de chauffe réduit. En effet, il est beaucoup plus rapide de chauffer parallèlement avec plusieurs résistances chauffantes de petites quantités de médicament contenues dans plusieurs cavités de réception, qu'une quantité de médicament plus importante correspondant à la somme de ces quantités avec une seule résistance chauffante dans une unique cavité de réception de contenance supérieure. Le dispositif permet ainsi de limiter le temps de chauffe à quelques minutes seulement (2 à 3 minutes par exemple pour quatre cavités de réception).

Un autre avantage de ce mode de réalisation est aussi de permettre à des médicaments difficilement solubles d'être administrés en quantité suffisante pour être efficace.

Avantageusement, dans ce cas, les cavités de réception du plateau de chauffe et les cheminées traversantes du réservoir peuvent être réparties radialement par rapport à l'arbre vertical avec un écartement angulaire constant. Le déplacement angulaire du réservoir est ainsi identique pour atteindre successivement chacune des positions de distribution, une position d'inhalation étant intercalée entre chaque position de distribution.

Avantageusement, l'ensemble peut comprendre en outre au moins un joint annulaire, disposé à l'interface entre le plateau de chauffe et la paroi inférieure du réservoir (le joint étant de préférence fixé sur le plateau de chauffe), qui entoure l'ouverture supérieure de la cavité de réception en position de distribution.

En position de distribution, l'étanchéité entre le réservoir et le plateau de chauffe est ainsi améliorée autour de l'ouverture de la cavité de réception.

Selon les variantes, ce joint annulaire peut être placé sur la paroi inférieure du réservoir rotatif, autour de l'orifice de distribution, ou plus préférentiellement sur la paroi supérieure du plateau de chauffe, autour de l'ouverture de la cavité de réception.

Avantageusement, le dispositif médical peut comprendre en outre une chambre de recueil de l'aérosol, par exemple tronconique ou en forme d'entonnoir inversé, située au-dessus du logement, qui recueille l'aérosol s'élevant à travers l'ensemble des cheminées traversantes et qui le guide vers l'interface d'inhalation.

Cette chambre permet ainsi de recueillir et de rassembler toutes les fractions d'aérosol formées dans les différentes cavités de réception du plateau de chauffe, qui s'élèvent des différentes cheminées, et de les guider vers l'interface d'inhalation pour qu'elles soient toutes inhalées par le patient.

Avantageusement, l'élément d'obturation peut comprendre un piston qui comporte une tige avec une extrémité inférieure sur laquelle est fixée un bouchon et une extrémité supérieure qui dépasse à l'extérieur du réservoir. Ce piston coulisse dans un conduit cylindrique à l'intérieur du réservoir entre une position basse, dans laquelle il est rappelé par un moyen de rappel élastique et dans laquelle le bouchon est plaqué contre l'orifice de distribution, et une position haute, dans laquelle l'orifice de distribution est libéré, obtenue en tirant vers le haut l'extrémité supérieure de la tige.

Ces moyens permettent avantageusement d'ouvrir, de refermer automatiquement et de maintenir fermer l'orifice de distribution du réservoir rotatif.

Avantageusement, le bâti peut comporter un bord circulaire s'étendant au-dessus du logement, qui comprend un cran saillant vers le haut situé radialement au niveau de chaque cavité de réception. Le réservoir peut alors comporter en outre un levier, sur lequel est fixé l'extrémité supérieure de la tige du piston, et dont une extrémité est en appui glissant sur ledit bord circulaire, de sorte que, lors du pivotement du réservoir, l'extrémité du levier se soulève à chaque cran, tirant vers le haut l'extrémité supérieure de la tige du piston.

Ces moyens permettent avantageusement d'ouvrir automatiquement l'élément d'obturation afin de libérer l'orifice de distribution lorsque le réservoir atteint une position de distribution et de le refermer automatiquement pour reboucher l'orifice de distribution dès que le réservoir quitte cette position de distribution.

En effet, lorsque le réservoir atteint une position de distribution, l'extrémité du levier qui est en appui glissant sur le bord circulaire atteint le cran saillant qui est situé radialement au niveau de la cavité de réception correspondante. En montant sur ce cran, l'extrémité du levier se soulève et tire vers le haut l'extrémité supérieure de la tige du piston qui y est fixée. Le piston coulisse alors dans son cylindre vertical et le bouchon qui est fixé à l'autre extrémité de sa tige s'écarte de l'orifice de distribution qui est ainsi ouvert.

Lorsque le réservoir se remet à tourner et quitte la position de distribution, l'extrémité du levier, qui est en appui glissant sur le bord circulaire, redescend du cran saillant. Le moyen de rappel élastique replace alors le piston en position basse et plaque à nouveau le bouchon contre l'orifice de distribution qui est ainsi refermé.

Avantageusement, le réservoir peut comprendre en outre une paroi latérale cylindrique à partir de laquelle s'étend au moins une nervure saillante latéralement en direction de l'extérieur, qui monte et descend alternativement de manière à former une rampe de hauteur variable sensiblement en forme de vague, ou à partir de laquelle s'étendent plusieurs paires de picots de guidage saillants latéralement en direction de l'extérieur et disposés l'un au-dessus de l'autre à la même hauteur dans chaque paire.

Le bâti peut lui comprendre une paroi latérale cylindrique à partir de laquelle s'étendent respectivement plusieurs paires de picots de guidage saillants latéralement en direction de l'intérieur et disposés l'un au-dessus de l'autre à la même hauteur dans chaque paire, ou à partir de laquelle s'étend au moins une nervure saillante latéralement en direction de l'intérieur, qui monte et descend alternativement de manière à former une rampe de hauteur variable sensiblement en forme de vague.

Ladite nervure peut alors coulisser entre les picots de guidage de chaque paire lors du pivotement du réservoir.

De cette façon, comme la nervure de hauteur variable coulisse entre les picots de hauteur fixe lorsque le réservoir pivote, la hauteur et l'inclinaison relative du réservoir par rapport au bâti change automatiquement selon sa position angulaire en fonction de la hauteur de la rampe à ce niveau.

La forme de la nervure peut ainsi être avantageusement prévue pour, lorsque le réservoir atteint une position de distribution, abaisser la partie du réservoir proche de l'orifice de distribution afin d'améliorer l'étanchéité entre le réservoir et le plateau de chauffe en comprimant le joint annulaire qui entoure l'ouverture de la cavité de réception si le dispositif en est pourvu, et pour incliner le réservoir afin de faciliter l'écoulement du médicament liquide à travers l'orifice de distribution.

La forme de cette nervure peut également être avantageusement prévue pour remonter le réservoir lorsqu'il n'est pas en position de distribution, afin de l'éloigner du plateau chauffant et ainsi de faciliter sa rotation.

Avantageusement, le bâti peut comprendre une coque intermédiaire servant de support pour le plateau de chauffe et renfermant le logement pour le réservoir. Le bord circulaire et la paroi latérale cylindrique peuvent alors avantageusement appartenir à la coque intermédiaire.

Cette coque intermédiaire sert ainsi de support pour tout le système de distribution et contient les crans saillants et les paires de picots de guidage ou la nervure qui coopèrent respectivement avec le levier et la nervure ou les picots de guidage du réservoir.

Avantageusement, un évent peut être ménagé dans une paroi de chaque cavité de réception, cet évent étant obturé temporairement par un élément de recouvrement, préférentiellement en silicone, qui est maintenu en appui contre l'évent par un électroaimant en position non-alimentée de l'électroaimant et qui est éloigné de l'évent en position alimentée de l'électroaimant.

Cet évent permet de faciliter l'écoulement du médicament liquide hors du réservoir pour pouvoir remplir la cavité de réception qui est étanche lorsque le dispositif est en position de distribution.

L'électroaimant permet d'ouvrir cet évent en écartant l'élément de recouvrement pour déclencher le remplissage et de le refermer par la suite en appuyant l'élément de recouvrement contre l'évent pour éviter une fuite de médicament liquide à ce niveau.

De plus, comme c'est en position non-alimentée de l'électroaimant que l'élément de recouvrement est maintenu en appui contre l'évent, la sécurité contre les fuites est améliorée.

Alternativement, le dispositif peut ne pas comporter d'électro-aimant, l'évent pouvant être ouvert et/ou fermé par tout autre type de mécanisme approprié.

Alternativement encore, l'évent peut être ouvert en permanence, le temps en position de distribution étant paramétré précisément pour obtenir le remplissage souhaité en évitant les fuites.

### BREVE DESCRIPTION DES FIGURES

D'autres avantages et caractéristiques de l'invention apparaîtront à la lecture de la description qui suit, illustrée par les figures dans lesquelles :
La figure 1 est une vue générale en perspective d'un exemple d'ensemble médical selon l'invention.
La figure 2 est une vue en coupe longitudinale de l'ensemble de la figure 1.
La figure 3 est une vue en perspective du plateau de chauffe de l'ensemble de la figure 1.
La figure 4 est une portion d'une vue en coupe selon l'axe IV-IV de la figure 3 illustrant plus particulièrement une cavité de réception.
La figure 5 et la figure 6 sont des vues en perspective, respectivement de dessus et de dessous, du réservoir amovible de l'ensemble de la figure 1.
La figure 7 et la figure 8 sont des vues en coupe du réservoir amovible des figures 5 et 6, réalisées au niveau de l'orifice de distribution et de son élément d'obturation, qui, respectivement, ferme ou laisse ouvert l'orifice de distribution.
La figure 9 est une vue en perspective de la coque intermédiaire de l'ensemble de la figure 1.
La figure 10 et la figure 11 sont des vues, respectivement de dessus et en coupe longitudinale, de la coque intermédiaire, du plateau de chauffe et du réservoir de l'ensemble de la figure 1, en position de distribution du réservoir.
La figure 12 et la figure 13 sont des vues, respectivement de dessus et de côté en perspective, du plateau de chauffe et du réservoir de l'ensemble de la figure 1, en position d'inhalation.
La figure 14 et la figure 15 sont des vues de dessous en perspective du plateau de chauffe de l'ensemble de la figure 1, illustrant plus particulièrement l'évent d'une cavité de réception respectivement fermé ou ouvert au moyen d'un élément de recouvrement en silicone et d'un électroaimant.

### DESCRIPTION DETAILLEE

Sur les figures, on a représenté un exemple de réalisation d'un ensemble 1, comprenant un dispositif médical 2 et un réservoir 3 amovible, qui est décrit en détail ci-après afin d'illustrer les caractéristiques et les avantages de l'invention. Il est toutefois rappelé que l'invention ne se limite pas à cet exemple.

Sauf précision contraire, un même élément apparaissant sur différentes figures présente une référence unique. Pour des questions de lisibilité, les échelles de taille entre éléments représentés ne sont pas forcément respectées.

Dans la présente demande, le terme « médicament » est employé pour désigner un médicament sous une forme liquide. Ce médicament, appelé aussi solution médicamenteuse, peut contenir un ou plusieurs principes actifs et un ou plusieurs excipients, liquides ou en solution.

Par convention dans cette demande, on définira les termes « haut », « bas », « supérieur », « inférieur », « dessus », « dessous » « horizontal » et « vertical » en fonction de l'orientation adoptée par les éléments sur les différentes figures, cette orientation étant prévue pour être celles de ces éléments en utilisation.

De même, les adjectifs « interne » ou « externe » et « intérieur » ou « extérieur » sont définis par rapport à la pièce considérée.

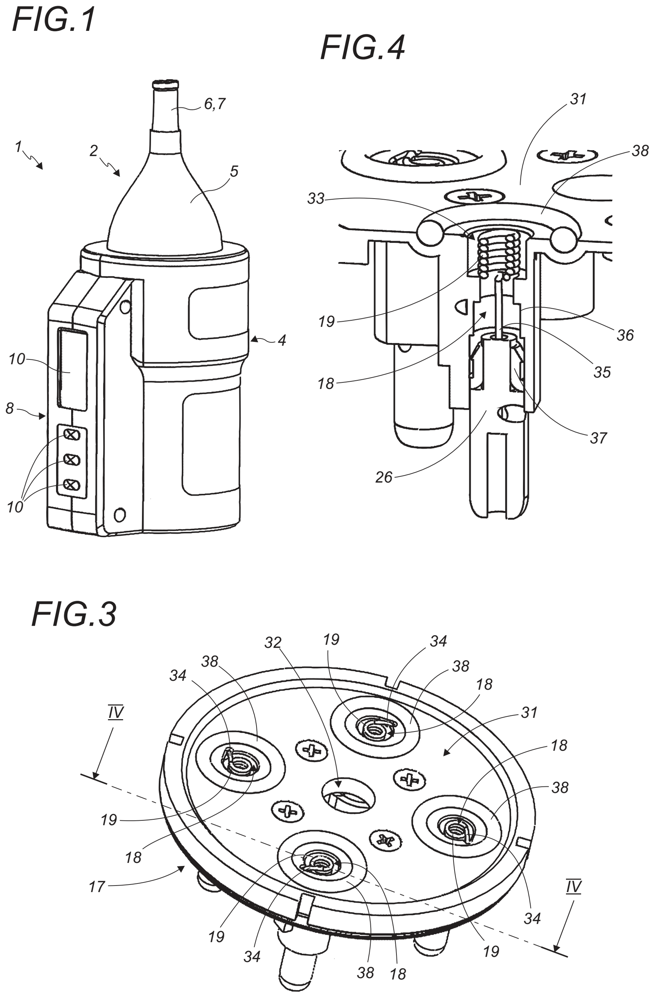

Sur la figure 1, l'ensemble 1 est vu de l'extérieur.

Le dispositif médical 2 comporte un bâti 4 renfermant des éléments fonctionnels du dispositif, prolongé en partie supérieure par une coiffe 5 sensiblement en forme d'entonnoir inversé (c'est-à-dire une structure avec une base inférieure large se rétrécissant progressivement vers le haut) et se termine par une interface d'inhalation 6 permettant au patient d'inhaler l'aérosol.

L'interface d'inhalation 6 représentée est un embout buccal 7, par exemple similaire aux embouts des inhalateurs buccaux traditionnels. Selon les variantes, il peut s'agir alternativement d'un embout nasal ou d'un masque permettant d'inhaler l'aérosol à la fois par le nez et la bouche.

Le dispositif médical 2 représenté est également équipé d'une interface de visualisation et de commande 8, qui sert d'interface entre le patient et le dispositif, et qui comprend par exemple un écran d'affichage 9 et plusieurs boutons 10, dont par exemple un bouton d'allumage (ON/OFF) permettant au patient de commander l'aérosolisation du médicament et l'extinction du dispositif (qui peut également ou alternativement être automatique) et/ou des boutons de navigation.

L'écran d'affichage 9 peut afficher différentes données, par exemple des données relatives à la date et l'heure de prise du médicament, au nom du médicament, à sa date de péremption, à la température de chauffe des résistances, au nombre d'utilisations ou de doses possibles ou restantes du médicament, à des indications thérapeutiques ou techniques, etc.

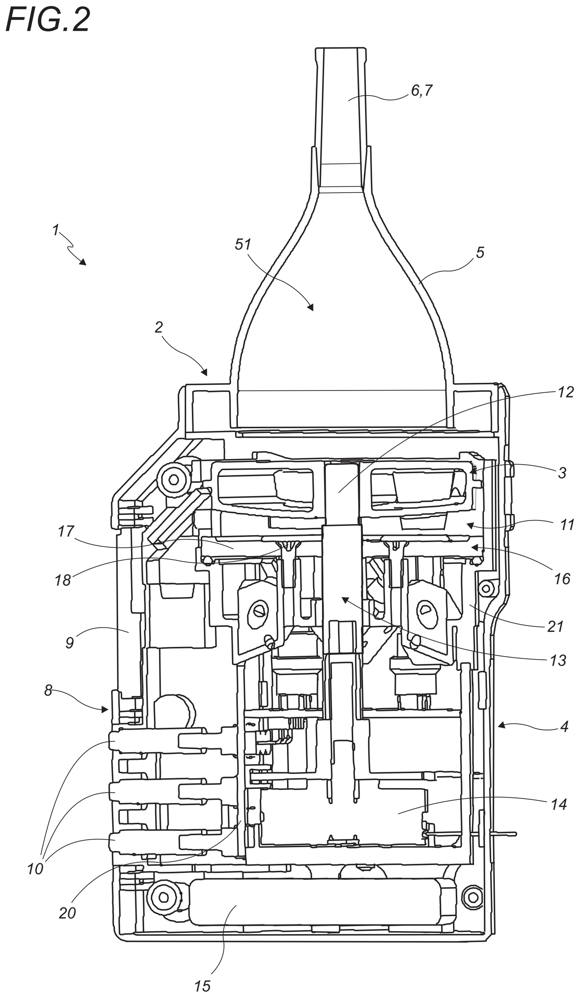

Sur la figure 2, l'ensemble 1 est représenté en coupe ce qui permet de visualiser ses éléments fonctionnels internes. Pour une meilleure compréhension, les éléments importants sont également représentés séparément sur les figures suivantes.

Comme visible sur cette figure, le bâti 4 renferme un logement 11 destiné à recevoir le réservoir 3, dans lequel débouche l'extrémité supérieure 12 d'un arbre 13 qui s'étend verticalement, de préférence sensiblement au centre du bâti 4.

Cet arbre 13 est entrainé en rotation par un moteur 14, alimenté par une batterie 15 préférentiellement rechargeable.

Le dispositif médical 2 comprend également un dispositif d'aérosolisation 16, comprenant un plateau de chauffe 17, également logé dans le bâti 4, sous le logement 11. Ce plateau de chauffe 17 comprend une ou plusieurs cavités de réception 18, équipées chacune d'une résistance chauffante 19. Ces résistances chauffantes 19 sont également alimentées par la batterie 15 et sont agencées de manière à pouvoir élever la température du contenu de la cavité de réception 18 correspondante lorsqu'elles fonctionnent.

Une unité de commande 20, également alimentée par la batterie 15, contrôle le fonctionnement de l'ensemble du dispositif médical 2 et commande en particulier la rotation de l'arbre vertical 13 et l'allumage des résistances chauffantes 19. Cette unité de commande 20 comprend par exemple une carte de circuit imprimé (couramment appelée carte PCB signifiant « Printed Circuit Board » en anglais) sur laquelle sont assemblés des composants électroniques.

Le bâti 14 comprend en outre une coque intermédiaire 21, servant de support au plateau de chauffe 17 et contenant en partie supérieure le logement 11 pour le réservoir 3.

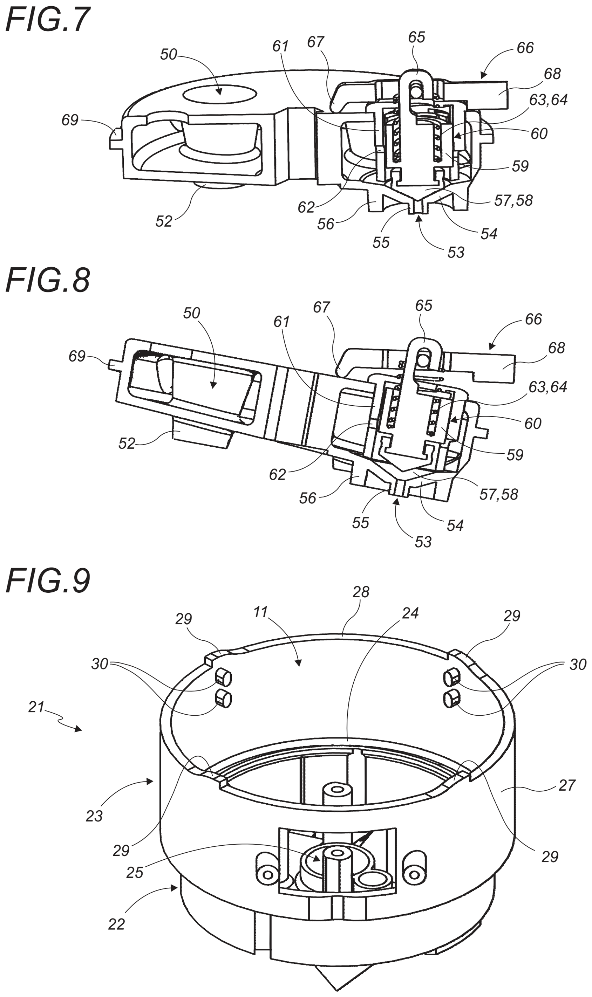

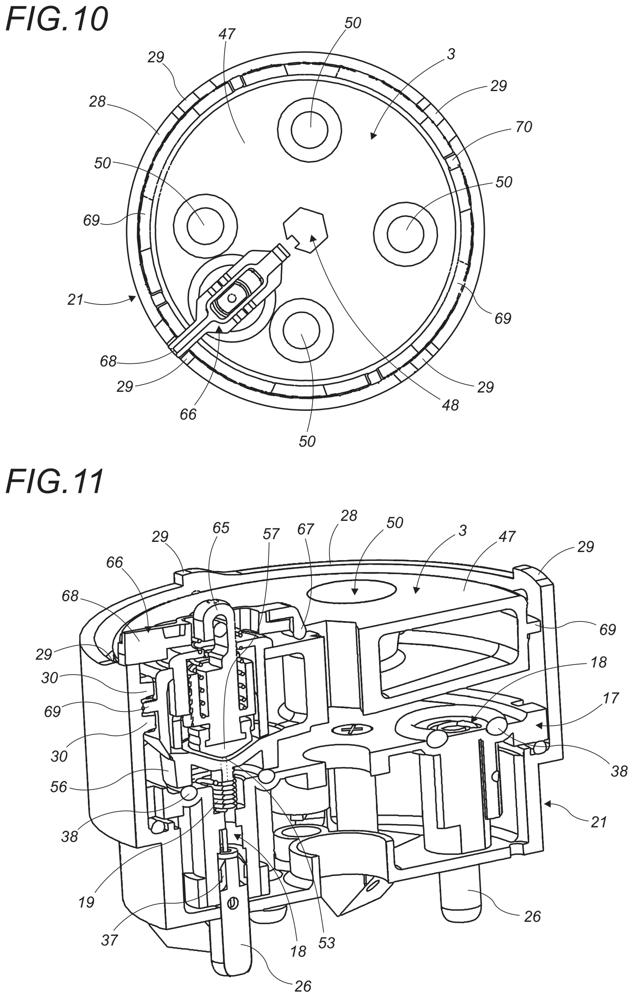

Cette coque intermédiaire a été représentée seule sur la figure 9 et avec le plateau de chauffe 17 et le réservoir 3 sur la figure 11.

Elle comporte une partie inférieure 22 sensiblement cylindrique, qui se poursuit par une partie supérieure 23 également sensiblement cylindrique mais de diamètre supérieur, ces deux parties 22, 23 étant réunies par un épaulement circulaire 24 sur lequel repose le plateau de chauffe 17.

La partie inférieure 22 présente une ouverture centrale 25 autorisant le passage de l'arbre 13 et abrite les éléments constitutifs du plateau de chauffe 17, tels que les cavités de réception 18, les résistances chauffantes 19 et leurs cosses de connexion 26.

La partie supérieure 23 renferme le logement 11 et présente une face supérieure ouverte permettant l'engagement et le retrait du réservoir 3.

Elle comporte également une paroi latérale cylindrique 27, terminée en extrémité supérieure par un bord circulaire 28 qui présente plusieurs crans 29 dépassant vers le haut et de préférence répartis radialement avec un écartement angulaire constant par rapport au centre du bord circulaire (quatre crans avec un écartement angulaire de 90° sur l'exemple représenté). Ces crans 29 saillants forment une rampe avec une montée progressive d'un côté, suivie d'une descente abrute de l'autre.

La paroi latérale cylindrique 27 comporte également sur sa face intérieure, des paires de picots de guidage 30, disposés l'un au-dessus de l'autre et s'étendant vers l'intérieur du logement 11, qui sont situées à une hauteur constante les unes par rapport aux autres. Une paire de picots de guidage 30 est disposée au niveau de chacun des crans 29.

Le plateau de chauffe a été représenté seul sur les figures 3 et 4. Il comporte une platine circulaire 31, également percée d'une ouverture centrale 32 pour permettre le passage de l'arbre 13, et dans laquelle sont ménagées les cavités de réception 18. Celles-ci sont au nombre de quatre sur l'exemple représenté, disposées radialement autour de l'ouverture centrale 32 avec un écartement angulaire constant de 90°.

Les cavités de réception 18 sont des chambres creuses étanches, de forme sensiblement cylindrique, comportant une ouverture supérieure 33 permettant leur remplissage par le médicament s'écoulant du réservoir 3. Leur volume intérieur libre est fixe et défini précisément. Il correspond par exemple à une contenance comprise entre 50 et 150 µL, et de préférence égale à 100 µL.

Chacune des cavités de réception 18 contient une résistance chauffante 19 par exemple sous la forme d'un serpentin, dont une première extrémité 34 est connectée à la platine circulaire 31 qui est électriquement conductrice et qui constitue une première borne pour la résistance 19, et dont la deuxième extrémité 35 plonge dans la cavité de réception 18 sans en toucher la paroi 36 et s'engage dans une cosse de connexion 26 débouchant en fond de cavité 18. Cette cosse de connexion 26 est isolée électriquement de la paroi 36 par un joint 37 en matériau électriquement isolant et résistant à la chaleur, et constitue une deuxième borne de signe opposé pour la résistance 19.

Ainsi disposées, les résistances chauffantes 19 sont capables de chauffer le médicament liquide contenu dans la cavité de réception 18 pour provoquer son aérosolisation. Elles peuvent être mises en route simultanément ou indépendamment les unes des autres, ce qui permet de chauffer simultanément un nombre quelconque de cavités 18 et ainsi de varier la quantité de médicament aérosolisé.

Le plateau de chauffe 17 comporte également des joints annulaires 38, par exemple toriques, fixés sur la platine 31 autour de l'ouverture 33 de chacune des cavités de réception 18, qui assurent l'étanchéité entre le réservoir et la cavité de réception 18 lors de la distribution du médicament. Alternativement, un joint annulaire pourrait être fixé sur le réservoir, autour de son orifice de distribution.

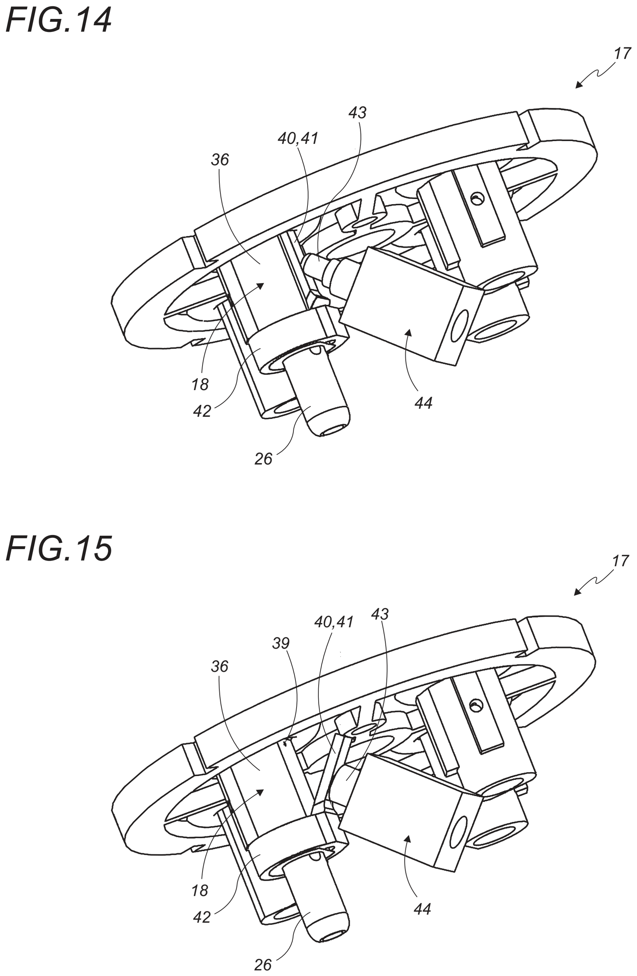

Comme représenté sur les figures 14 et 15, chaque cavité de réception 18 comporte en outre un évent 39 percé dans sa paroi 36, qui permet l'écoulement du médicament dans la cavité de réception 18, malgré le caractère étanche du réservoir 3, de la cavité 18 et de leur jonction.

Cet évent 39 est obturable par un élément de recouvrement 40, préférentiellement en silicone, qui se présente par exemple sous la forme d'une languette 41, articulée à une bague 42 qui est enfilée autour et à l'extérieur de la cavité de réception 18.

Cette languette 41 est fixée à la tige 43 d'un électroaimant 44 qui la maintient en appui contre l'évent 39 de préférence lorsque l'électroaimant n'est pas alimenté et qui l'écarte de l'évent 39 en se rétractant lorsque l'électroaimant 44 est alimentée.

L'unité de commande 20 pilote l'alimentation de l'électroaimant 44 et déclenche l'ouverture de l'évent 39 uniquement lorsqu'un écoulement du médicament est souhaité en position de distribution de l'ensemble 1.

Bien qu'un seul électro-aimant 44 ait été représenté sur les figures 14 et 15, un système équivalent existe pour chacune des cavités de réception 18.

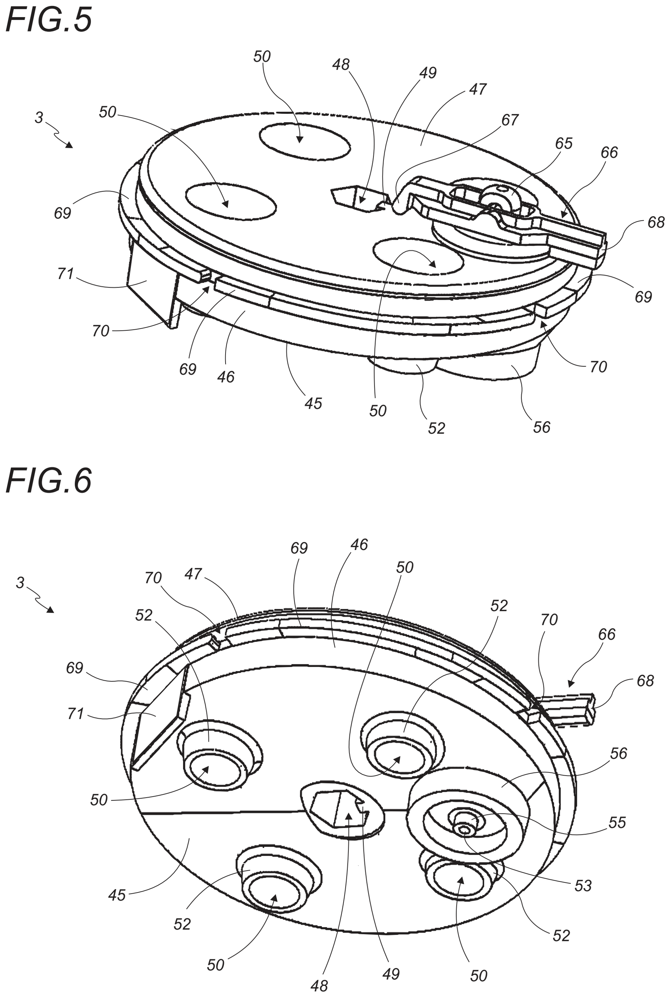

En plus du dispositif médical 2, l'ensemble 1 selon l'invention comprend un réservoir 3, qui est représenté seul sur les figures 5 à 8.

Le réservoir 3 est un élément hermétique, amovible, indépendant du dispositif médical 2, qui contient le médicament à administrer au patient. Il s'agit d'un élément consommable, commercialisé séparément du dispositif médical 2. Le dispositif médical 2, en lui-même, ne contient pas de médicament.

Le réservoir 3 est préalablement rempli de médicament et scellé par un professionnel (du domaine médical ou pharmaceutique) de sorte que le patient ne puisse avoir accès audit médicament. Bien qu'il n'ait pas accès au contenu du réservoir 3, le patient a accès au réservoir 3 en lui-même, qu'il peut monter dans ou démonter hors du logement 11.

Ce réservoir 3 est un récipient fermé, de forme sensiblement cylindrique, comprenant une paroi inférieure 45, une paroi latérale 46 cylindrique et une paroi supérieure 47.

Il comporte une ouverture centrale traversante 48 (ou alternativement un renfoncement dans sa paroi inférieure 45), permettant d'y engager l'extrémité 12 de l'arbre 13 et qui présente une forme, par exemple hexagonale, complémentaire de celle de l'extrémité 12 permettant à l'arbre 13 d'entrainer le réservoir 3 en rotation. Avantageusement, une nervure d'indexation 49 s'étend verticalement à l'intérieur de l'ouverture 48 et sert à la fois de détrompeur pour garantir une bonne position de montage pour le réservoir 3 et de languette anti-rotation pour s'opposer aux mouvements relatifs entre le réservoir 3 et l'arbre 13.

Le réservoir 3 comprend également des cheminées 50, qui traversent la paroi inférieure 45 et la paroi supérieure 47, et dont le nombre et l'emplacement correspondent à ceux des cavités de réception 18, c'est-à-dire qu'elles sont agencées de manière à se retrouver chacune juste au-dessus de l'ouverture supérieure 33 de l'une des cavités de réception 18 dans une position particulière du réservoir, dite « position d'inhalation », obtenue lors de sa rotation.

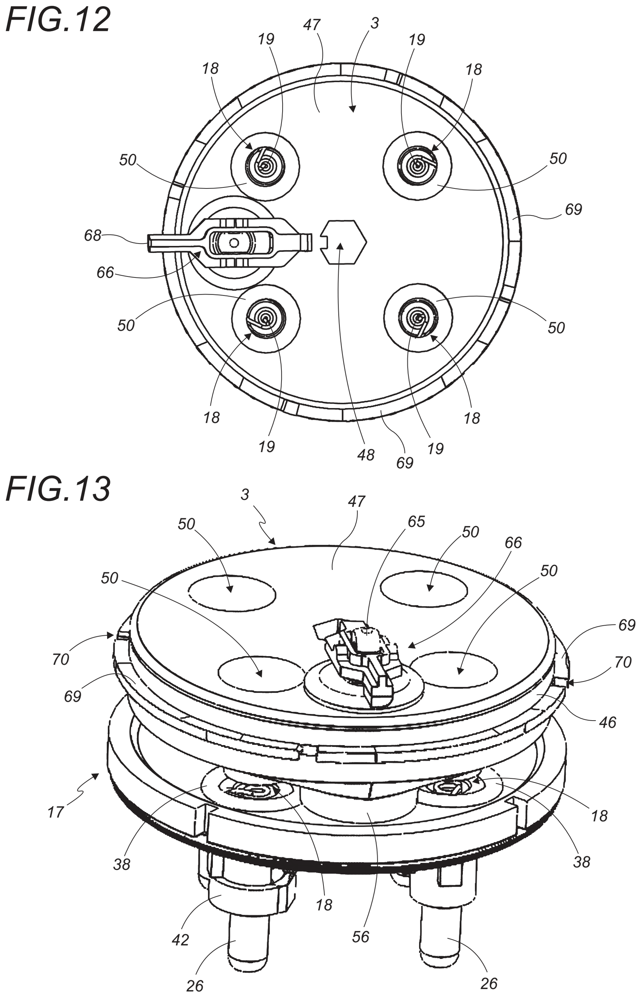

Dans cette position illustrée sur les figures 12 et 13, les cavités de réception 18 se retrouvent en communication fluidique, via les cheminées 50, avec une chambre de recueil 51 de l'aérosol à l'intérieur de la coiffe 5 qui communique avec l'embout buccal 7.

Si les cavités de réception 18 et les cheminées sont disposées radialement autour de l'arbre 13 avec un écartement angulaire constant, notamment comme sur l'exemple représenté, il y a alors plusieurs positions d'inhalation qui sont atteintes successivement par une rotation du réservoir d'un angle correspondant à cet écart angulaire.

Afin de mieux canaliser l'aérosol s'échappant des cavités de réception 18 en position d'inhalation, les cheminées 50 peuvent avantageusement être prolongées vers le bas par une collerette 52.

Le réservoir 3 comporte en outre un orifice de distribution 53 ménagé dans sa paroi inférieure 45 et disposé de manière à se retrouver juste au-dessus de l'ouverture supérieure 33 de chacune des cavités de réception 18 lorsque le réservoir 3 se trouve après rotation dans l'une des positions particulières appelées « positions de distribution ».

L'orifice 53 est préférentiellement entouré d'une portion de paroi conique 54 en pente vers l'orifice 53 et prolongée par un embout cylindrique 55, l'ensemble étant similaire au « cône » d'une seringue pour faciliter l'écoulement des très petites quantités de médicament, surtout lorsqu'il présente une certaine viscosité.

L'orifice de distribution 53 est également entouré d'une collerette 56 s'étendant vers le bas, prévue pour venir en appui contre le joint annulaire 38 correspondant lorsque le réservoir est dans une position de distribution comme illustré sur les figures 10 et 11.

Un élément d'obturation 57 bouche l'orifice de distribution 53 lorsque le réservoir 3 n'est pas en position de distribution. Il s'agit sur l'exemple représenté d'un bouchon 58 en matériau souple et de forme conique complémentaire à celle de la portion de paroi 54, qui est monté à l'extrémité inférieure de la tige 59 d'un piston 60 coulissant dans un conduit cylindrique 61 vertical présent dans le réservoir 3 et présentant des ouvertures 62 pour permettre l'entrée du médicament.

Un moyen de rappel élastique 63, par exemple un ressort 64, pousse la tige 59 vers le bas et plaque le bouchon 58 contre l'orifice de distribution 53.

L'extrémité supérieure 65 de la tige 59 traverse la paroi supérieure 47 et peut coulisser de manière étanche à travers celle-ci. Elle est fixée à la partie médiane d'un levier 66 qui s'étend radialement au-dessus de la paroi supérieure 47.

Le levier 66 comporte une première extrémité 67, dirigée vers l'ouverture centrale 48, qui est en contact avec la paroi supérieure 47 et sert de point d'appui de basculement pour le levier 66, et une deuxième extrémité 68 qui dépasse latéralement au-delà de la paroi latérale 46.

Lorsque le réservoir 3 est placé dans le logement 11 de la coque intermédiaire 21, la deuxième extrémité 68 du levier 66 repose sur le bord circulaire 58 (visible sur la figure 11) et coulisse sur celui-ci lorsque le réservoir pivote. Lorsque le réservoir arrive dans une position de distribution, l'extrémité 68 franchit un cran 29, ce qui fait basculer le levier 66 et tire vers le haut l'extrémité supérieure 65 de la tige 59, libérant ainsi l'orifice de distribution 53 du bouchon 54.

Lorsque le réservoir quitte cette position de distribution, l'extrémité 68 descend brusquement du cran 29 et l'extrémité supérieure 65 de la tige 59 est immédiatement poussée vers le bas par le ressort 64, ce qui rebouche l'orifice de distribution 53.

Le réservoir 3 comprend en outre une nervure 69 en forme de vague, c'est-à-dire une nervure dont le profil présente des ondulations successives avec une alternance de creux et de bosses et de portions montantes et descendante, qui s'étend latéralement depuis la paroi latérale 46 en direction de l'extérieur et en fait le tour.

Cette nervure 69 est située à une hauteur lui permettant d'être placée entre les picots de guidage 30 de chaque paire de la coque intermédiaire 21 lorsque le réservoir 3 est dans le logement 11 comme illustré sur la figure 11. Cette nervure 69 permet ainsi de modifier la hauteur relative et l'inclinaison du réservoir 3 par rapport à la coque intermédiaire 21 selon la position angulaire du réservoir pendant sa rotation.

Des interruptions 70 sont prévues dans la nervure 69 pour permettre la mise en place ou le retrait du réservoir 3 dans ou hors du logement 11 malgré la présence des picots de guidage 30, leur positionnement étant coordonné avec celui de la nervure d'indexation 49 pour se trouver au niveau des picots de guidage lors de l'engagement du réservoir.

Selon un autre mode de réalisation, la nervure 69 et les picots de guidage 30 peuvent être inversés, la nervure 69 étant portée par la coque intermédiaire 21 et les picots de guidage par le réservoir 3.

Le réservoir 3 peut en outre comporter une étiquette 71 présentant des informations sur le médicament qu'il contient, par exemple sous la forme d'un code barre ou d'un code QR pouvant être lu et exploité par l'unité de commande 20.

## Revendications

1. Ensemble (1) comprenant un dispositif médical (2) d'administration d'un médicament par inhalation et un réservoir (3) hermétique amovible contenant ledit médicament sous une forme liquide, ledit dispositif médical (2) générant un aérosol inhalable par le patient par élévation de la température du médicament, et comprenant :
- un bâti (4) renfermant un logement (11) dans lequel est placé le réservoir (3),
- une interface d'inhalation (6) de l'aérosol,
- une unité de commande (20), contrôlant le fonctionnement du dispositif médical (2),
- un arbre vertical (13), dont une extrémité (12) dépasse dans le logement (11) et sur laquelle le réservoir (3) est monté,
- un moteur (14) qui entraine en rotation l'arbre vertical (13) et qui, par l'intermédiaire de cet arbre vertical (13), fait pivoter le réservoir (3) entre au moins une position de distribution et au moins une position d'inhalation,
- un plateau de chauffe (17), disposé sous le logement (11), qui comprend au moins une cavité de réception (18) du médicament, comportant une ouverture supérieure (33) et équipée d'une résistance chauffante (19) permettant d'élever la température à l'intérieur de la cavité de réception (18),
et le réservoir (3) comportant :
- une paroi inférieure (45) dans laquelle est ménagée un orifice de distribution (53), permettant l'écoulement du médicament hors du réservoir (3), ledit orifice de distribution (53) se trouvant au-dessus de l'ouverture supérieure (33) de la cavité de réception (18) lorsque le réservoir (3) est en position de distribution,
- un élément d'obturation (57), qui bouche l'orifice de distribution (53) lorsque le réservoir (3) n'est pas en position de distribution et le laisse libre lorsque le réservoir (3) est en position de distribution, et
- au moins une cheminée traversante (50), qui, lorsque le réservoir (3) est en position d'inhalation, se trouve au-dessus de l'ouverture supérieure (33) de la cavité de réception (18), mettant la cavité de réception (18) en communication fluidique avec l'interface d'inhalation (6).

2. Ensemble (1) selon la revendication 1, **caractérisé en ce que** le plateau de chauffe (17) comprend plusieurs, préférentiellement quatre, cavités de réception (18), équipées chacune d'une résistance chauffante (19) ; et **en ce que** le réservoir (3) comporte un nombre de cheminées traversantes (50) correspondant au nombre de cavités de réception (18), lesdites cheminées traversantes (50) se trouvant chacune au-dessus de l'ouverture supérieure (33) de l'une des cavités de réception (18) lorsque le réservoir (3) est en position d'inhalation, mettant ladite cavité de réception (18) en communication fluidique avec l'interface d'inhalation (6).

3. Ensemble (1) selon la revendication 2, **caractérisé en ce que** les cavités de réception (18) du plateau de chauffe (17) et les cheminées traversantes (50) du réservoir (3) sont réparties radialement par rapport à l'arbre vertical (13) avec un écartement angulaire constant.

4. Ensemble (1) selon l'une des revendications précédentes, **caractérisé en ce qu'**il comprend en outre au moins un joint annulaire (38), disposé à l'interface entre le plateau de chauffe (17) et la paroi inférieure (45) du réservoir (3), et de préférence fixé sur le plateau de chauffe (17), qui entoure l'ouverture supérieure (33) de la cavité de réception (18) en position de distribution.

5. Ensemble (1) selon l'une des revendications précédentes, **caractérisé en ce que** le dispositif médical (2) comprend en outre une chambre de recueil (51) de l'aérosol, située au-dessus du logement (11), qui recueille l'aérosol s'élevant à travers l'ensemble des cheminées traversantes (50) et qui le guide vers l'interface d'inhalation (6).

6. Ensemble (1) selon l'une des revendications précédentes, **caractérisé en ce que** l'élément d'obturation (57) comprend un piston (60) qui comporte une tige (59) avec une extrémité inférieure sur laquelle est fixée un bouchon (58) et une extrémité supérieure (65) qui dépasse à l'extérieur du réservoir (3), ledit piston (60) coulissant dans un conduit cylindrique (61) à l'intérieur du réservoir (3) entre une position basse, dans laquelle il est rappelé par un moyen de rappel élastique (63) et dans laquelle le bouchon (58) est plaqué contre l'orifice de distribution (53), et une position haute, dans laquelle l'orifice de distribution (53) est libéré, obtenue en tirant vers le haut l'extrémité supérieure (65) de la tige (59).

7. Ensemble (1) selon la revendication précédente, **caractérisé en ce que** le bâti (4) comporte un bord circulaire (28) s'étendant au-dessus du logement (11), qui comprend un cran (29) saillant vers le haut situé radialement au niveau de chaque cavité de réception (18), et **en ce que** le réservoir (3) comporte en outre un levier (66), sur lequel est fixé l'extrémité supérieure (65) de la tige (59) du piston (60), et dont une extrémité (68) est en appui glissant sur ledit bord circulaire (28), de sorte que, lors du pivotement du réservoir (3), l'extrémité (68) du levier (66) se soulève à chaque cran (29), tirant vers le haut l'extrémité supérieure (65) de la tige (59) du piston (60).

8. Ensemble (1) selon l'une des revendications précédentes, **caractérisé en ce que** le réservoir (3) comprend en outre une paroi latérale (46) cylindrique à partir de laquelle s'étend au moins une nervure (69) saillante latéralement en direction de l'extérieur, qui monte et descend alternativement de manière à former une rampe de hauteur variable sensiblement en forme de vague, ou à partir de laquelle s'étendent plusieurs paires de picots de guidage (30) saillants latéralement en direction de l'extérieur et disposés l'un au-dessus de l'autre à la même hauteur dans chaque paire ; **en ce que** le bâti (4) comprend une paroi latérale cylindrique (27) à partir de laquelle s'étendent respectivement plusieurs paires de picots de guidage (30) saillants latéralement en direction de l'intérieur et disposés l'un au-dessus de l'autre à la même hauteur dans chaque paire, ou à partir de laquelle s'étend au moins une nervure (69) saillante latéralement en direction de l'intérieur, qui monte et descend alternativement de manière à former une rampe de hauteur variable sensiblement en forme de vague, et **en ce que** ladite nervure (69) coulisse entre les picots de guidage (30) de chaque paire lors du pivotement du réservoir (3).

9. Ensemble (1) selon les revendications 7 et 8, **caractérisé en ce que** le bâti (4) comprend une coque intermédiaire (21) servant de support pour le plateau de chauffe (17) et renfermant le logement (11) pour le réservoir (3), et **en ce que** ledit bord circulaire (28) et ladite paroi latérale cylindrique (27) appartiennent à la coque intermédiaire (21).

10. Ensemble (1) selon l'une des revendications précédentes, **caractérisé en ce qu'**un évent (39) est ménagé dans une paroi (36) de chaque cavité de réception (18), cet évent (39) étant obturé temporairement par un élément de recouvrement (40), qui est maintenu en appui contre l'évent (39) par un électroaimant (44) en position non-alimentée de l'électroaimant (44) et qui est éloigné de l'évent (39) en position alimentée de l'électroaimant (44).
